# EUROPEAN PATENT APPLICATION

(11) **EP 4 667 472 A1**
(43) Date of publication of application: **24.12.2025**
(21) Application number: 24756731.6
(22) Date of filing: 06.02.2024
(51) Int. Cl.: C07D 493/04, C08K 5/06, C08K 5/053, C08L 23/10

(54) **COMPOUND, COMPOSITION, MOLDED ARTICLE, AND PRODUCTION METHOD**

(30) Priority: 17.02.2023 JP 2023023452
(71) Applicant: MITSUBISHI GAS CHEMICAL COMPANY, INC., Chiyoda-ku Tokyo 100-8324 (JP)
(72) Inventor: MASUYA, Yoshihiro, Kurashiki-shi, Okayama 712-8525 (JP); FUJISAWA, Minori, Kurashiki-shi, Okayama 712-8525 (JP); MATSUDA, Daiki, Kurashiki-shi, Okayama 712-8525 (JP); UTAMURA, Tatsuya, Kurashiki-shi, Okayama 712-8525 (JP); NISHIUCHI, Junya, Kurashiki-shi, Okayama 712-8525 (JP)
(74) Representative: Müller-Boré & Partner Patentanwälte PartG mbB
(86) International application number: PCT/JP2024/003823
(87) International publication number: WO 2024/171881

(57) **Abstract**

A compound represented by the following formula (I): 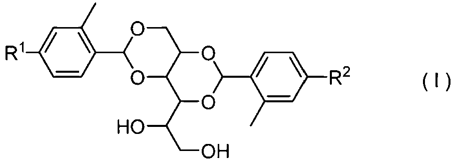 wherein R¹ and R² each independently represent an alkyl group having 2 to 5 carbon atoms.

## Description

### Technical Field

The present invention relates to a compound, a composition, a formed article, and a production method.

### Background Art

As additives that impart excellent optical properties to polymer resins, nucleating agents are known. As the nucleating agents, dibenzylidene sorbitol compounds are known.

Patent Literature 1 discloses a method for modifying a crystalline plastic, including adding dibenzylidene sorbitol to a polymer or a copolymer of a crystalline plastic and thermoforming the resultant.

Patent Literature 2 discloses a method for modifying polypropylene, including adding 1·3,2·4-di(methylbenzylidene) sorbitol to crystalline polypropylene or a copolymer thereof and thermoforming the resultant.

Patent Literature 3 discloses that a resin composition having transparency can be obtained by applying 1,3:2,4-bis(O-methylbenzylidene) sorbitol.

Patent Literature 4 discloses bis(3,4-dialkylbenzylidene) sorbitol acetal that is useful as a nucleating agent and is a plastic additive particularly useful for improving optical properties of polymer materials.

Patent Literature 5 discloses a nucleating agent or a clarifying agent exhibiting one or more of improved transparency, yellowing, and organoleptics.

### Citation List

### Patent Literature

Patent Literature 1: Japanese Patent Laid-Open No. 51-22740
Patent Literature 2: Japanese Patent Publication No. 55-12460
Patent Literature 3: Japanese Patent Laid-Open No. 7-286066
Patent Literature 4: Japanese Patent Publication No. 7-121947
Patent Literature 5: Japanese Translation of PCT International Application Publication No. 2009-534465

### Summary of Invention

### Technical Problem

However, in recent years, further enhancement in various properties of nucleating agents or clarifying agents has been much in demand.

In view of the foregoing, an object of the present invention is to provide a new compound that is suitable for nucleating agents or clarifying agents.

### Solution to Problem

As a result of intensive studies, the present inventors have found a new compound that is suitable for nucleating agents or clarifying agents, thereby completing the present invention.

That is, the present invention is as follows.
[1] A compound represented by the following formula (I): 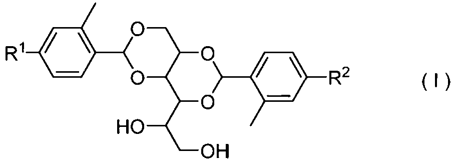 wherein R¹ and R² each independently represent an alkyl group having 2 to 5 carbon atoms.
[2] The compound according to [1], wherein R¹ and R² each independently represent ethyl, n-propyl, i-propyl, n-butyl, or i-butyl.
[3] A composition comprising the compound according to [1] or [2].
[4] The composition according to [3], wherein the composition is used as a nucleating agent or a clarifying agent.
[5] A formed article obtained by mixing the compound according to [1] or [2] or the composition according to [3] or [4], and a polyolefin resin.
[6] A method for producing a transparent formed article, comprising mixing the compound according to [1] or [2] or the composition according to [3] or [4], and a polyolefin resin.
[7] A method for producing a compound represented by the following formula (I), comprising reacting a benzaldehyde (α) having an alkyl group having 2 to 5 carbon atoms located at a para position and a methyl group located at an ortho position with a polyol in the presence of an acid catalyst: 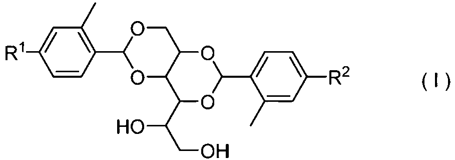 wherein R¹ and R² each independently represent an alkyl group having 2 to 5 carbon atoms.
[8] The production method according to [7], further comprising reacting a benzaldehyde (β) having an alkyl group having 2 to 5 carbon atoms located at an ortho position and a methyl group located at a para position,
wherein a content of the benzaldehyde (β) based on a total amount of the benzaldehyde (α) and the benzaldehyde (β) is 50 mass% or less.

### Advantageous Effect of Invention

According to the present invention, a new compound that is suitable for nucleating agents or clarifying agents can be provided.

### Description of Embodiments

Embodiments of the present invention will hereinafter be described in detail. The present invention is not limited to the embodiments described below, and various modifications could be made within the scope of the gist of the present invention to exploit the present invention.

### <Compound>

An embodiment of the present invention relates to a compound represented by the following formula (I) (hereinafter also referred to as "compound (I)"): 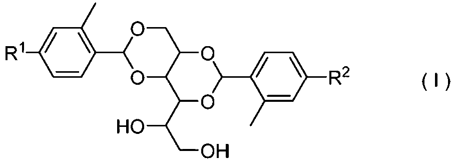 wherein R¹ and R² each independently represent an alkyl group having 2 to 5 carbon atoms.

In the formula (I), R¹ and R² each independently represent an alkyl group having 2 to 5 carbon atoms. In the present specification, the alkyl group having 2 to 5 carbon atoms is not particularly limited, and examples thereof include ethyl, n-propyl, i-propyl, n-butyl, i-butyl, t-butyl, sec-butyl, n-pentyl, i-pentyl, neopentyl, t-pentyl, sec-pentyl, and 1,2-dimethylpropyl.

In the formula (I), R¹ and R² each independently represent an alkyl group having 2 to 5 carbon atoms, and more preferably represent an alkyl group having 2 to 4 carbon atoms from the viewpoint that such an alkyl group has a high 5% weight loss temperature. R¹ and R² are not particularly limited, and each independently preferably represent ethyl, n-propyl, i-propyl, n-butyl, or i-butyl, and more preferably represent n-propyl, i-propyl, n-butyl, or i-butyl from the same viewpoint. In the present specification, "5% weight loss temperature" means a temperature at which the percentage of weight loss caused by heating under an atmosphere of inert gas reaches within 5% with respect to the weight before heating.

Examples of performance required for nucleating agents or clarifying agents (hereinafter also generally simply referred to as "nucleating agent or the like") include a high 5% weight loss temperature. In general, when a compound used for a nucleating agent or the like has a high 5% weight loss temperature, the nucleating agent or the like is less likely to transpire and/or decompose even under high temperature, and occurrence of odor derived from the nucleating agent or the like can be suppressed when a formed article is produced or when the formed article is processed at high temperature. In the formula (I), when R¹ and R² each independently represent an alkyl group having 2 to 5 carbon atoms, the 5% weight loss temperature is high, whereby the compound is more suitable for a nucleating agent or the like. The present inventors suppose that the reason thereof is as follows, although it is not intended to particularly limit the present invention thereby. That is, in the formula (I), when R¹ and R² each independently represent an alkyl group having 1 carbon atom, the 5% weight loss temperature tends to be low. In the formula (I), when R¹ and R² each independently represent an alkyl group having 6 or more carbon atoms, the 5% weight loss temperature tends to be high, but fine crystals of the compound (I) tend not to deposit when a formed article is produced, whereby the compound (I) can not exhibit performance as a nucleating agent.

300°C or more of the 5% weight loss temperature of a nucleating agent or the like is general performance as a nucleating agent or the like and, therefore, the 5% weight loss temperature of the compound (I) is preferably 300°C or more, more preferably 310°C or more, and particularly preferably 315°C or more, although the temperature is not particularly limited. The 5% weight loss temperature can be measured based on the method described in Examples described later.

In the formula (I), from the viewpoint that homogeneous dissolution and/or diffusion is possible in a polyolefin resin at low melt-kneading temperature (for example, 200°C), R¹ and R² are not particularly limited, and each independently preferably represent a n-propyl group, an i-propyl group, a n-butyl group, or an i-butyl group, more preferably represent an i-propyl group or an i-butyl group, and particularly preferably represent a compound (A) or a compound (B).

In the formula (I), R¹ and R² may be the same or different, but are preferably the same from the viewpoint of easy production of the compound (I) and the performance as a nucleating agent or the like.

The compound (I) of the present embodiment is not particularly limited, and examples thereof include compounds represented by the following formulas (A), (B), (C), (D), and (E) (hereinafter each also referred to as "compound (A)", "compound (B)", "compound (C)", "compound (D)", and "compound (E)").

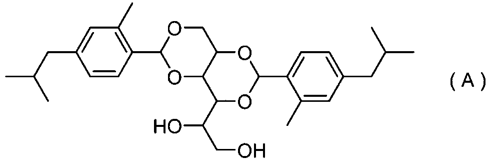

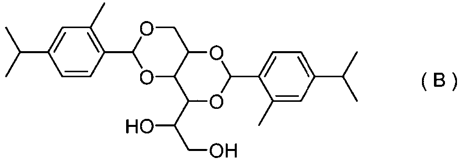

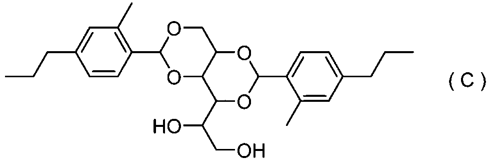

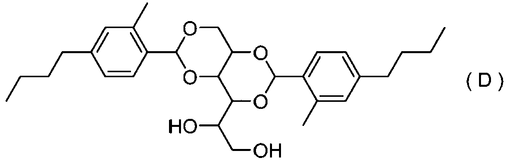

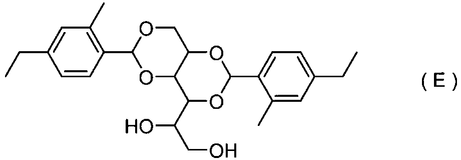

The compound (I) of the present embodiment is not particularly limited, and can be suitably used as a nucleating agent or a clarifying agent.

When there is a stereoisomer of the compound (I) of the present embodiment (for example, an enantiomer), the compound (I) is intended to encompass each stereoisomer and mixtures thereof (for example, a racemate).

### <Composition>

An embodiment of the present invention relates to a composition containing the compound (I). The composition of the present embodiment contains at least the compound (I). The composition of the present embodiment may be composed of the compound (I) alone, or may contain other components. The composition of the present embodiment includes an aspect in which one or more compounds selected from the compounds (A) to (E) are contained.

The composition of the present embodiment is not particularly limited, and can be suitably used as a nucleating agent or a clarifying agent. By using the composition of the present embodiment as a nucleating agent or a clarifying agent, excellent optical properties can be imparted to a formed article described later.

The composition of the present embodiment is not particularly limited, and may be, for example, in the form of powder, particulates, or liquid, and the composition is preferably in a form that is excellent as a nucleating agent or the like.

In the present embodiment, examples of other components contained in the composition include additives usually contained in a nucleating agent or the like, and examples thereof include acid inorganic additive components, hydrotalcites, inorganic silicon compounds, and other nucleating agents, although the other components are not particularly limited.

In the composition of the present embodiment, the content of the compound (I) based on the total amount of the composition is not particularly limited, and the content can be appropriately selected in accordance with the use of the composition. When the composition is used as a nucleating agent, the content of the compound (I) based on the total amount of the composition is not particularly limited as long as the composition can exhibit the performance as a nucleating agent.

### <Formed article>

An embodiment of the present invention relates to a formed article obtained by mixing the compound (I) and a polyolefin resin. Also, an embodiment of the present invention relates to a formed article obtained by mixing the composition containing the compound (I) and a polyolefin resin. The formed article of the present embodiment contains at least the compound (I) and the polyolefin resin, and can also be formed by using the composition containing the compound (I) as described above. Therefore, the formed article of the present embodiment may contain a component derived from the composition of the present embodiment.

When the compound (I) of the present embodiment is a soluble nucleating agent or the like, the compound (I) homogeneously dissolved or dispersed in the polyolefin resin by heating can impart excellent optical properties to the formed article. Therefore, the formed article of the present embodiment is preferably obtained by heating and mixing the compound (I) and the polyolefin resin and forming the resultant, and is more preferably obtained by melt-kneading the compound (I) and the polyolefin resin and forming the resultant. That is, in the present embodiment, aspects of the formed article obtained by mixing the compound (I) and the polyolefin resin include a formed article obtained by melt-kneading the compound (I) and the polyolefin resin, and a formed article obtained by forming the compound (I) and the polyolefin resin.

In the present embodiment, by mixing the compound (I) and the polyolefin resin, the formed article can have excellent optical properties. The excellent optical properties are not particularly limited, and examples thereof include transparency, non-yellowing properties, and glossiness. When the formed article of the present embodiment has one or more properties selected from transparency, non-yellowing properties, and glossiness, the compound (I) is suitable for a nucleating agent or the like.

The compound (I) of the present embodiment can impart transparency to a formed article obtained by mixing the compound (I) and the polyolefin resin. The transparency can be evaluated by, for example, the haze value. Preferred haze values depend on the polyolefin resin used and the use thereof, and for example, when polypropylene is used, mixing the polypropylene with the compound (I) can provide a formed article having a haze value of 14% or less, and can further provide a formed article having a haze value of 11% or less. The haze value can be measured based on the method described in Examples described later.

The compound (I) of the present embodiment can suppress yellowing of the formed article obtained by mixing the compound (I) and the polyolefin resin. Yellowing of the formed article can be evaluated by yellowing index (YI). The yellowing index (YI) of the formed article is not particularly limited, and is preferably 3 or less. When the yellowing index (YI) of the formed article is more than 3, the yellowing index (YI) may be adjusted with a pigment such as a blue coloring agent. The yellowing index (YI) can be measured based on the method described in Examples described later.

In the present specification, the polyolefin resin is not particularly limited, and examples thereof include polyethylene resins and polypropylene resins, and specific examples include high density polyethylene, medium density polyethylene, straight-chain polyethylene, ethylene copolymers having an ethylene content of 50 mass% or more, propylene homopolymer, propylene copolymers having a propylene content of 50 mass% or more, butene homopolymer, butene copolymers having a butene content of 50 mass% or more, methylpentene homopolymer, methylpentene copolymers having a methylpentene content of 50 mass% or more, and polybutadiene. The copolymer is not particularly limited, and examples thereof include random copolymers and block copolymers. The stereoregularity of the polyolefin resin is not particularly limited, and examples thereof include isotactic stereoregularity and syndiotactic stereoregularity.

In the present embodiment, the polyolefin resin is not particularly limited, and is preferably polypropylene, more preferably a random copolymer of ethylene and polypropylene, and particularly preferably a polypropylene having an ethylene content of 0.5 mass% or more and 5 mass% or less from the viewpoint of enhancing the transparency of the formed article. As the polyolefin resin, one type thereof may be used alone, or two or more types thereof may be used in combination.

The formed article of the present embodiment may further contain other components. The other components contained in the formed article are not particularly limited, and examples thereof include phenol antioxidants, phosphorus antioxidants, sulfur antioxidants, oxidation inhibitors, fatty acid metal salts, ultraviolet absorbents, hindered amine compounds, flame retardants, flame-retardant auxiliaries, lubricants, fillers, hydrotalcites, antistatic agents, fluorescent whitening agents, pigments, dyes, plasticizers, light stabilizers, colorants, and other nucleating agents. As the other components, one type thereof may be used alone, or two or more types thereof may be used in combination.

In the formed article of the present embodiment, the content of the compound (I) is preferably 0.005 mass% or more and 5 mass% or less, more preferably 0.01 mass% or more and 2.5 mass% or less, and particularly preferably 0.02 mass% or more and 1 mass % or less based on the total amount of the polyolefin resin. With the content of the compound (I) falling within the above range, transparency can be imparted to the formed article. In the formed article, if the content of the compound (I) is more than 5 mass% based on the total amount of the polyolefin resin, defects in appearance of the formed article tend to occur. In the formed article, if the content of the compound (I) is less than 0.005 mass% based on the total amount of the polyolefin resin, fine crystals of the compound (I) tend not to deposit when the formed article is produced, whereby the compound can not exhibit performance as a nucleating agent.

In general, with a large content of the nucleating agent or the like, the nucleating agent or the like tends not to be homogeneously dissolved and/or dispersed in the polyolefin resin, whereby fish eyes or the like tend to occur, resulting in occurrence of defects in appearance of the formed article. Therefore, a nucleating agent or the like that can prevent defects in appearance of the formed article even when the content of the nucleating agent or the like is large is in demand. The compound (I) of the present embodiment tends to have high solubility and/or dispersibility in the polyolefin resin and, therefore, the content of the compound (I) can be larger than that of conventional products. The present inventors suppose that the reason thereof is as follows, although it is not intended to particularly limit the present invention thereby. That is, it is considered that, in the formula (I), R¹ and R² each independently represent an alkyl group having 2 to 5 carbon atoms, whereby the solubility of the compound (I) in the polyolefin resin increases, and, accordingly, the compound (I) is easily homogeneously dissolved and/or dispersed in the polyolefin resin. In the formula (I), when R¹ and R² each independently represent an alkyl group having 1 carbon atom, the solubility of the compound (I) in the polyolefin resin tends not to increase. In the formula (I), when R¹ and R² each independently represent an alkyl group having 6 or more carbon atoms, the solubility of the compound (I) in the polyolefin resin tends to increase, but fine crystals of the compound (I) tend not to deposit when the formed article is produced, whereby the compound (I) can not exhibit performance as a nucleating agent. The content of the compound (I) can be determined by the charging ratio at the time of preparation of a polyolefin resin composition described later, or by conventionally known analytical and analysis methods formed articles. The methods for analyzing formed articles are not particularly limited, and examples thereof include extraction methods such as a Soxhlet extraction method, an ultrasonic extraction method, and a high-speed high-pressure extraction method; or a method in which a solution in which a nucleating agent or the like is dissolved obtained by a dissolution/reprecipitation method or the like is analyzed by high performance liquid chromatography (HPLC) or the like.

In the formed article of the present embodiment, the total sum of the content of the polyolefin resin and the content of the compound (I) is preferably 50 mass% or more and 100 mass% or less, more preferably 65 mass% or more and 100 mass% or less, and particularly preferably 70 mass% or more and 100 mass% or less based on the total amount of the formed article from the viewpoint of enhancing the strength and the flexibility of the formed article.

### <Method for producing formed article>

An embodiment of the present invention relates to a method for producing a transparent formed article, including mixing the compound (I) or the composition containing the compound, and the polyolefin resin. The method for producing the formed article of the present embodiment is not particularly limited as long as the formed article may be obtained by the method, and it is preferred that the formed article is produced by the following method. In the method for producing the formed article of the present embodiment, the phrase "mixing the compound (I) (or the composition containing the compound) and the polyolefin resin" may mean, for example, merely mixing the compound (I) and the polyolefin resin in the step of preparing a polyolefin resin composition described later, or mixing the compound (I) and the polyolefin resin by melt-kneading or the like in the step of melt-kneading described later.

In the method for producing the formed article of the present embodiment, by mixing the compound (I), a formed article excellent in the optical properties can be produced, and a transparent formed article can be produced. It can be evaluated that the formed article is transparent by the method described above.

### (Preparation of polyolefin resin composition)

The method for producing the formed article of the present embodiment can include a step of preparing a polyolefin resin composition containing the compound (I) (or the composition containing the compound) and the polyolefin resin. The polyolefin resin composition contains at least the compound (I) and the polyolefin resin, and can also be prepared by using the composition containing the compound (I) as described above. Therefore, the polyolefin resin composition may contain a component derived from the composition of the present embodiment. The compound (I) (or the composition containing the compound) and the polyolefin resin that can be used here are as described above. The other components contained in the formed article described above may be added to the polyolefin resin composition.

When the polyolefin resin composition is melt-kneaded, a predetermined amount of the compound (I) (or the composition containing the compound) and the polyolefin resin may be directly charged into a melt-kneader without separately providing a step of preparing the polyolefin resin composition.

### (Melt-kneading of the polyolefin resin composition)

The method for producing the formed article of the present embodiment can include a step of melt-kneading the polyolefin resin composition. Examples of the method for melt-kneading the polyolefin resin composition include a melt-kneading method using a conventionally known melt-kneading apparatus.

In the present embodiment, the melt-kneading temperature is not particularly limited, and is, for example, preferably 180°C or more and 230°C or less, and more preferably 190°C or more and 210°C or less. With the melt-kneading temperature falling within the above range, the starting material can be sufficiently dissolved, and various problems that occur when the melt-kneading temperature is high described later can be suppressed. The melt-kneading temperature can be adjusted by, for example, using a melt-kneading apparatus that may adjust the temperature.

In general, with a melt-kneading temperature, problems such as deterioration in the formed article, yellowing of the formed article, and generation of odor due to thermal decomposition of a nucleating agent or the like tend to occur. On the other hand, with a low melt-kneading temperature, the nucleating agent or the like tends not to be homogeneously dissolved and/or dispersed in the polyolefin resin, whereby fish eyes or the like tend to occur, resulting in occurrence of defects in appearance of the formed article. Therefore, a nucleating agent or the like having satisfactory solubility and/or dispersibility in the polyolefin resin at a low melt-kneading temperature is in demand. The compound (I) of the present embodiment tends to be able to be homogeneously dissolved and/or dispersed in the polyolefin resin even at a low melt-kneading temperature (for example, 200°C). The present inventors suppose that the reason thereof is as follows, although it is not intended to particularly limit the present invention thereby. That is, it is considered that, in the formula (I), R¹ and R² each independently represent an alkyl group having 2 to 5 carbon atoms, whereby the solubility of the compound (I) in the polyolefin resin increases, and, accordingly, the compound (I) is easily homogeneously dissolved and/or dispersed in the polyolefin resin. In the formula (I), when R¹ and R² each independently represent an alkyl group having 1 carbon atom, the solubility of the compound (I) in the polyolefin resin tends not to increase. In the formula (I), when R¹ and R² each independently represent an alkyl group having 6 or more carbon atoms, the solubility of the compound (I) in the polyolefin resin tends to increase, but fine crystals of the compound (I) tend not to deposit when the formed article is produced. It can be evaluated that the compound (I) tends to be able to be homogeneously dissolved and/or dispersed in the polyolefin resin by occurrence of fish eyes, and the fish eyes can be measured based on the method described in Examples described later.

In the present embodiment, the melt-kneading speed and the melt-kneading time are not particularly limited, and can be appropriately selected.

### (Formation of polyolefin resin composition)

The method for producing the formed article of the present embodiment can include a step of forming a polyolefin resin composition. As a method for forming the polyolefin resin composition, for example, a conventionally known forming method can be used. In the step of forming the polyolefin resin composition, the melt-kneaded polyolefin resin composition may be cooled to be cured. The forming method is not particularly limited, and examples thereof include injection-molding, extrusion blow molding, injection blow molding, stretch blow molding, compression molding, rotational molding, profile extrusion, plate extrusion, thermoforming, film extrusion, film forming, and stretched film extrusion.

When the melt-kneaded polyolefin resin composition is cooled, fine crystals of the compound (I) deposit, whereby the compound (I) can exhibit sufficient performance as a nucleating agent.

### <Method for producing compound>

An embodiment of the present invention relates to a method for producing the compound represented by the formula (I). In the method for producing the compound (I) of the present embodiment, it is preferred to react a benzaldehyde (α) having an alkyl group having 2 to 5 carbon atoms located at the para position and a methyl group located at the ortho position with a polyol in the presence of an acid catalyst.

The compound (I) of the present embodiment can be produced by, for example, the production method described in Japanese Translation of PCT International Application Publication No. 2009-534465.

In the method for producing the compound (I) of the present embodiment, the alkyl group having 2 to 5 carbon atoms located at the para position in the benzaldehyde (α) is not particularly limited, and the alkyl group is preferably ethyl, n-propyl, i-propyl, n-butyl, or i-butyl. A conventionally known compound can be used as the benzaldehyde (α).

R¹ and R² in the formula (I) each correspond to the alkyl group having 2 to 5 carbon atoms located at the para position of the benzaldehyde (α). That is, the compound (I) having desired R¹ and R² can be produced by using a benzaldehyde (α) in which the alkyl group having 2 to 5 carbon atoms located at the para position is the desired R¹ or R². For the formula (I) in which R¹ and R² are the same, one benzaldehyde (α) having an alkyl group having 2 to 5 carbon atoms located at the para position may be used, and for the formula (I) in which R¹ and R² are different from each other, two benzaldehydes (α) having different alkyl groups having 2 to 5 carbon atoms located at the para position can be used to produce the compound (I).

In the method for producing the compound (I) of the present embodiment, a benzaldehyde (β) having an alkyl group having 2 to 5 carbon atoms located at the ortho position and a methyl group located at the para position may be further reacted with the polyol. The benzaldehyde (β) may be a benzaldehyde formed as a byproduct in the production of the benzaldehyde (α). The content of the benzaldehyde (β) based on the total amount of the benzaldehyde (α) and the benzaldehyde (β) is not particularly limited, and the content is preferably 50 mass% or less, and more preferably 30 mass% or less from the viewpoint of the yield of the compound (I).

When the benzaldehyde (β) is further reacted in the method for producing the compound (I) of the present embodiment, a compound derived from the benzaldehyde (β) may be produced. The compound derived from the benzaldehyde (β) is not particularly limited, and examples thereof include a compound in which R¹ and/or R² is located at the ortho position and the methyl group is located at the para position in the formula (I). The compound derived from the benzaldehyde (β) may or may not be removed by conventionally known purification methods, but it is preferred that the compound derived from the benzaldehyde (β) is removed.

In the method for producing the compound (I) of the present embodiment, the polyol is not particularly limited, and examples thereof include sorbitol. As the polyol, an optically active substance may be used, or a racemate may be used.

In the method for producing the compound (I) of the present embodiment, the acid catalyst is not particularly limited, and examples thereof include organic acid catalysts and inorganic acid catalysts. Of these, organic acid catalysts are preferred, and sulfonic acids such as p-toluenesulfonic acid are more preferred.

In the method for producing the compound (I) of the present embodiment, the reaction between the benzaldehyde (α) and a polyol is preferably dehydration condensation. The dehydration condensation is not particularly limited, and it is preferred that the dehydration condensation is performed in water or/and a polar organic solvent. The polar organic solvent is not particularly limited, and alcohol organic solvents such as methanol are more preferred.

### Examples

The present embodiment will hereinafter be described more specifically with reference to Examples and Comparative Examples, but the present embodiment is not limited only to these Examples. The measurement method used in the present embodiment is as follows.

### [Melting point]

The exothermic peak temperature of the compound when the temperature was increased from room temperature at 10°C/minute was measured as the melting point (°C) by using a differential scanning calorimeter (SHIMADZU CORPORATION, DSC-60A Plus).

### <Production of compound and evaluation thereof>

### (Example 1)

### Synthesis of compound (A) (bis-1,3:2,4-(4'-isobutyl-2'-methylbenzylidene) sorbitol)

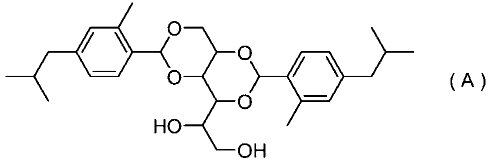

30.5 g of 4-isobutyl-2-methylbenzaldehyde (containing approximately 14% of 2-isobutyl-4-methylbenzaldehyde), 20.4 g of D-sorbitol, 27.5 g of p-toluenesulfonic acid monohydrate, 110.0 g of methanol, and 60.0 g of purified water were provided in a 500 mL reaction container provided with a stirrer and an entry for nitrogen. These materials were stirred at 30°C for 24 hours, and the obtained solid was collected by filtration and then was washed using purified water. An aqueous solution of potassium hydroxide was added to the washed white powder to adjust the pH thereof to more than 7, and then the resulting powder was washed at 60°C for 1 hour. The washed powder was collected by filtration and then was washed with methanol. Next, the powder was stirred in heptane at 70°C and then was filtered, which was then washed with methanol. The isolated white powder was dried in a vacuum oven to obtain 32.1 g of the compound (A). The melting point of the compound (A) was 215 to 216°C, and the 5% weight loss temperature was shown in Table 1. The purity was more than approximately 99% based on GC-MS (SHIMADZU CORPORATION, GCMS-QP2010Ultra).

¹H NMR (400 MHz, DMSO-d₆, ppm): 0.85 (d, 6H, ⁱBu), 0.85 (d, 6H, ⁱBu), 1.78-1.87 (m, 2H, ⁱBu), 2.33 (s, 3H, Me), 2.35 (s, 3H, Me), 2.40 (d, 4H, ⁱBu), 3.34-3.40 (m, 1H, sugar H), 3.52-3.57 (m, 1H, sugar H), 3.65-3.71 (m, 1H, sugar H), 3.81-3.84 (m, 1H, sugar H), 3.90 (s, 1H, sugar H), 4.06-4.20 (m, 3H, sugar H), 4.33 (t, 1H, - CH₂OH), 4.66 (d, 1H, -CHOH), 5.69 (s, 2H, acetal), 6.95-6.98 (m, 4H, aromatic H), 7.38-7.41 (m, 2H, aromatic H).

### (Example 2)

### Synthesis of compound (B) (bis-1,3:2,4-(4'-isopropyl-2'-methylbenzylidene) sorbitol)

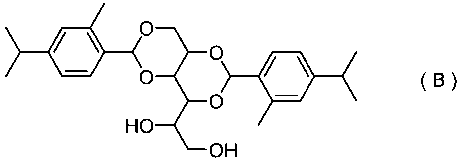

24.4 g of 4-isopropyl-2-methylbenzaldehyde (containing approximately 7% of 2-isopropyl-4-methylbenzaldehyde), 13.7 g of D-sorbitol, 22.1 g of p-toluenesulfonic acid monohydrate, 80.3 g of methanol, and 45.0 g of purified water were provided in a 500 mL reaction container provided with a stirrer and an entry for nitrogen. These materials were stirred at 30°C for 24 hours, and the obtained solid was collected by filtration and then was washed using purified water. An aqueous solution of potassium hydroxide was added to the washed white powder to adjust the pH thereof to more than 7, and then the resulting powder was washed at 60°C for 1 hour. The washed powder was collected by filtration and then was washed with methanol. Next, the powder was stirred in heptane at 70°C and then was filtered, which was then washed with methanol. The isolated white powder was dried in a vacuum oven to obtain 13.1 g of the compound (B). The melting point of the compound (B) was 189 to 190°C, and the 5% weight loss temperature was shown in Table 1. The purity was more than approximately 99% based on GC-MS.

¹H NMR (400 MHz, DMSO-d₆, ppm): 1.18 (s, 6H, ⁱPr), 1.20 (s, 6H, ⁱPr), 2.36 (s, 3H, Me), 2.38 (s, 3H, Me), 2.79-2.90 (m, 2H, ⁱPr), 3.35-3.40 (m, 1H, sugar H), 3.53-3.57 (m, 1H, sugar H), 3.67-3.70 (m, 1H, sugar H), 3.81-3.84 (m, 1H, sugar H), 3.91 (s, 1H, sugar H), 4.09-4.19 (m, 3H, sugar H), 4.32 (t, 1H, -CH₂OH), 4.65 (d, 1H, -CHOH), 5.70 (s, 2H, acetal), 7.04-7.06 (m, 4H, aromatic H), 7.39-7.42 (m, 2H, aromatic H).

### (Example 3)

### Synthesis of compound (C) (bis-1,3:2,4-(4'-n-propyl-2'-methylbenzylidene) sorbitol)

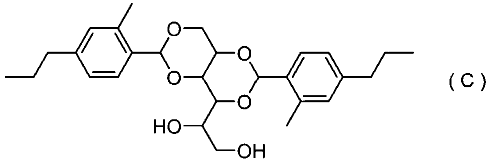

24.2 g of 4-n-propyl-2-methylbenzaldehyde (containing approximately 26% of 2-n-propyl-4-methylbenzaldehyde), 15.1 g of D-sorbitol, 19.8 g of p-toluenesulfonic acid monohydrate, 80.1 g of methanol, and 45.5 g of purified water were provided in a 500 mL reaction container provided with a stirrer and an entry for nitrogen. These materials were stirred at 30°C for 24 hours, and the obtained solid was collected by filtration and then was washed using purified water. An aqueous solution of potassium hydroxide was added to the washed white powder to adjust the pH thereof to more than 7, and then the resulting powder was washed at 60°C for 1 hour. The washed powder was collected by filtration and then was washed with methanol. Next, the powder was stirred in heptane at 70°C and then was filtered, which was then washed with methanol. The isolated white powder was dried in a vacuum oven to obtain 26.6 g of the compound (C). The melting point of the compound (C) was 239 to 240°C, and the 5% weight loss temperature was shown in Table 1. The purity was more than approximately 99% based on GC-MS.

¹H NMR (400 MHz, Acetone-d₆, ppm): 0.94 (t, 6H, ⁿPr), 1.59-1.68 (m, 4H, ⁿPr), 2.43 (s, 3H, Me), 2.44 (s, 3H, Me), 2.56 (t, 4H, ⁿPr), 3.43-4.28 (br, 10H, sugar H, -CH₂OH, -CHOH), 5.77 (s, 1H, acetal), 5.79 (s, 1H, acetal), 7.01-7.04 (m, 4H, aromatic H), 7.48-7.53 (m, 2H, aromatic H).

### (Example 4)

### Synthesis of compound (D) (bis-1,3:2,4-(4'-n-butyl-2'-methylbenzylidene) sorbitol)

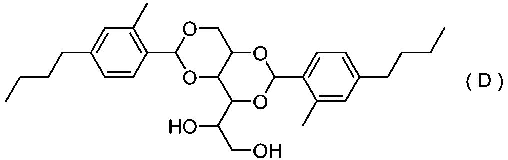

21.3 g of 4-n-butyl-2-methylbenzaldehyde (containing approximately 31% of 2-n-butyl-4-methylbenzaldehyde), 10.0 g of D-sorbitol, 16.3 g of p-toluenesulfonic acid monohydrate, 72.3 g of methanol, and 37.2 g of purified water were provided in a 500 mL reaction container provided with a stirrer and an entry for nitrogen. These materials were stirred at 40°C for 24 hours, and the obtained solid was collected by filtration and then was washed using purified water. An aqueous solution of potassium hydroxide was added to the washed white powder to adjust the pH thereof to more than 7, and then the resulting powder was washed at 60°C for 1 hour. The washed powder was collected by filtration and then was washed with methanol. Next, the powder was stirred in heptane at 70°C and then was filtered, which was then washed with methanol. The isolated white powder was dried in a vacuum oven to obtain 9.9 g of the compound (D). The melting point of the compound (D) was 238 to 239°C, and the 5% weight loss temperature was shown in Table 1. The purity was more than approximately 99% based on GC-MS.

¹H NMR (400 MHz, Acetone-d₆, ppm): 0.92 (t, 6H, ⁿBu), 1.30-1.40 (m, 4H, ⁿBu), 1.55-1.62 (m, 4H, ⁿBu), 2.41 (s, 3H, Me), 2.42 (s, 3H, Me), 2.57 (t, 4H, ⁿBu), 3.41-4.26 (br, 10H, sugar H, -CH₂OH, -CHOH), 5.75 (s, 1H, acetal), 5.77 (s, 1H, acetal), 7.00-7.02 (m, 4H, aromatic H), 7.46-7.51 (m, 2H, aromatic H).

### (Example 5)

### Synthesis of compound (E) (bis-1,3:2,4-(4'-ethyl-2'-methylbenzylidene) sorbitol)

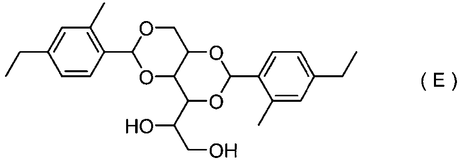

24.9 g of 4-ethyl-2-methylbenzaldehyde (containing approximately 31% of 2-ethyl-4-methylbenzaldehyde), 15.1 g of D-sorbitol, 26.2 g of p-toluenesulfonic acid monohydrate, 100.0 g of methanol, and 50.0 g of purified water were provided in a 500 mL reaction container provided with a stirrer and an entry for nitrogen. These materials were stirred at 30°C for 24 hours, and the obtained solid was collected by filtration and then was washed using purified water. An aqueous solution of potassium hydroxide was added to the washed white powder to adjust the pH thereof to more than 7, and then the resulting powder was washed at 60°C for 1 hour. The washed powder was collected by filtration and then was washed with methanol. Next, the powder was stirred in heptane at 70°C and then was filtered, which was then washed with methanol. The isolated white powder was dried in a vacuum oven to obtain 10.8 g of the compound (E). The melting point of the compound (E) was 232 to 233°C, and the purity was more than approximately 99% based on GC-MS.

¹H NMR (400 MHz, DMSO-d₆, ppm): 1.17 (t, 6H, Et), 2.35 (s, 3H, Me), 2.37 (s, 3H, Me), 2.53-2.59 (m, 4H, Et), 3.34-3.40 (m, 1H, sugar H), 3.52-3.57 (m, 1H, sugar H), 3.64-3.70 (m, 1H, sugar H), 3.81-3.84 (m, 1H, sugar H), 3.91 (s, 1H, sugar H), 4.10-4.19 (m, 3H, sugar H), 4.35 (t, 1H, -CH₂OH), 4.68 (d, 1H, -CHOH), 5.70 (s, 2H, acetal), 7.01-7.03 (m, 4H, aromatic H), 7.37-7.43 (m, 2H, aromatic H).

### (Comparative Example 1)

The 5% weight loss temperature of bis-1,3:2,4-(4'-methylbenzylidene) sorbitol (CAS RN: 81541120, hereinafter also referred to as "compound (X)") was measured. The result was shown in Table 1.

### (Comparative Example 2)

The 5% weight loss temperature of bis-1,3:2,4-(3',4'-dimethylbenzylidene) sorbitol (CAS RN: 135861562, hereinafter also referred to as "compound (Y)") was measured. The result was shown in Table 1.

### (Comparative Example A-1 to A-2)

The 5% weight loss temperature of each of the following compounds (A-1) and (A-2) was measured. The result was shown in Table 1.

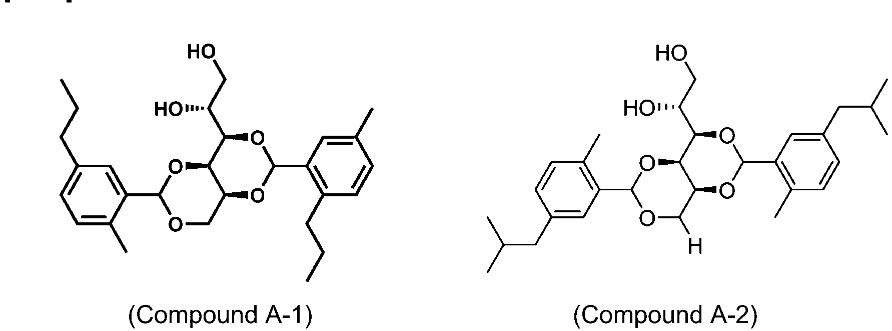

### [Measurement of 5% weight loss temperature]

The temperature was increased from room temperature at 10°C/minute under a nitrogen atmosphere and a temperature indicating loss of the weight of each compound was measured by using a thermogravimetry/differential thermal analysis simultaneous measurement apparatus (SHIMADZU CORPORATION, DTG-60A). The result was shown in the following Table. It was found from the Table that the 5% weight loss temperature of the compound of each Example was 315°C or more and therefore the compound was excellent in the thermal stability.

**[Table 1]**

| | Compound | 5% weight loss temperature (°C) |
|---|---|---|
| Example 1 | Compound (A) | 322 |
| Example 2 | Compound (B) | 315 |
| Example 3 | Compound (C) | 323 |
| Example 4 | Compound (D) | 322 |
| Comparative Example 1 | Compound (X) | 308 |
| Comparative Example 2 | Compound (Y) | 310 |
| Comparative Example A-1 | Compound (A-1) | 297 |
| Comparative Example A-2 | Compound (A-2) | 295 |

### <Production of formed article>

### (Example 6)

0.5 parts by mass of the compound (A) (bis-1,3:2,4-(4'-isobutyl-2'-methylbenzylidene) sorbitol), 0.05 parts by mass of tetrakis[methylene-3-(3,5-di-t-butyl-4-hydroxyphenyl)propionate]methane (CAS RN: 6683198), 0.1 parts by mass of Tris (2,4-di-t-butylphenyl)phosphite (CAS RN: 31570044), and 0.08 parts by mass of calcium stearate (CAS RN: 1592230) were added to 100 parts by mass of a polypropylene random copolymer resin (Japan Polypropylene Corporation, WINTEC PP WMG03) to prepare a polyolefin resin composition. The polyolefin resin composition (containing polypropylene) was melt-kneaded at 200°C at approximately 100 min⁻¹ for 3 minutes with a desktop kneader (Xplore Instruments BV, MC15HT). Next, the polyolefin resin composition at 200°C was fed into an injection-molding apparatus to produce a formed article having a diameter of 30.0 mm and a thickness of 1.0 mm.

### (Example 7)

0.8 parts by mass of the compound (A) (bis-1,3:2,4-(4'-isobutyl-2'-methylbenzylidene) sorbitol), 0.05 parts by mass of tetrakis[methylene-3-(3,5-di-t-butyl-4-hydroxyphenyl)propionate]methane (CAS RN: 6683198), 0.1 parts by mass of Tris (2,4-di-t-butylphenyl)phosphite (CAS RN: 31570044), and 0.08 parts by mass of calcium stearate (CAS RN: 1592230) were added to 100 parts by mass of a polypropylene random copolymer resin (Japan Polypropylene Corporation, WINTEC PP WMG03) to prepare a polyolefin resin composition. The polyolefin resin composition was melt-kneaded at 200°C at approximately 100 min⁻¹ for 3 minutes with a desktop kneader (Xplore Instruments BV, MC15HT). Next, the polyolefin resin composition at 200°C was fed into an injection-molding apparatus to produce a sheet-like formed article having a diameter of 30.0 mm and a thickness of 1.0 mm.

### (Example 8)

A formed article was produced in the same manner as in Example 6, except that 0.5 parts by mass of the compound (B) (bis-1,3:2,4-(4'-isopropyl-2'-methylbenzylidene) sorbitol) was used instead of the compound (A).

### (Example 9)

A formed article was produced in the same manner as in Example 7, except that 0.8 parts by mass of the compound (B) (bis-1,3:2,4-(4'-isopropyl-2'-methylbenzylidene) sorbitol) was used instead of the compound (A).

### (Example 10)

A formed article was produced in the same manner as in Example 6, except that 0.5 parts by mass of the compound (C) (bis-1,3:2,4-(4'-n-propyl-2'-methylbenzylidene) sorbitol) was used instead of the compound (A).

### (Example 11)

A formed article was produced in the same manner as in Example 6, except that 0.5 parts by mass of the compound (D) (bis-1,3:2,4-(4'-n-butyl-2'-methylbenzylidene) sorbitol) was used instead of the compound (A).

### (Example 12)

A formed article was produced in the same manner as in Example 6, except that 0.5 parts by mass of the compound (E) (bis-1,3:2,4-(4'-ethyl-2'-methylbenzylidene) sorbitol) was used instead of the compound (A).

### (Comparative Example 3)

A formed article was produced in the same manner as in Example 6, except that the compound (A) was not used.

### (Comparative Example 4)

A formed article was produced in the same manner as in Example 6, except that 0.2 parts by mass of the compound (X) (bis-1,3:2,4-(4'-methylbenzylidene) sorbitol) was used instead of the compound (A).

### (Comparative Example 5)

A formed article was produced in the same manner as in Example 6, except that 0.2 parts by mass of the compound (Y) (bis-1,3:2,4-(3',4'-dimethylbenzylidene) sorbitol) was used instead of the compound (A).

### (Comparative Example A-1-1)

A formed article was produced in the same manner as in Example 6, except that 0.5 parts by mass of the above compound (A-1) was used instead of the compound (A).

### (Comparative Example A-1-2)

A formed article was produced in the same manner as in Example 6, except that 0.8 parts by mass of the above compound (A-1) was used instead of the compound (A).

### (Comparative Example A-2-1)

A formed article was produced in the same manner as in Example 6, except that 0.5 parts by mass of the above compound (A-2) was used instead of the compound (A).

### (Comparative Example A-2-2)

A formed article was produced in the same manner as in Example 6, except that 0.8 parts by mass of the above compound (A-2) was used instead of the compound (A).

### (Comparative Example B-1-1)

A formed article was produced in the same manner as in Example 6, except that 0.5 parts by mass of the following compound (B-1) was used instead of the compound (A).

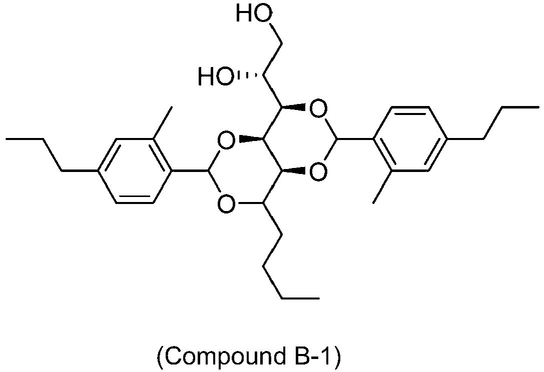

### (Comparative Example B-1-2)

A formed article was produced in the same manner as in Example 6, except that 0.8 parts by mass of the above compound (B-1) was used instead of the compound (A).

### (Comparative Example B-2-1)

A formed article was produced in the same manner as in Example 6, except that 0.5 parts by mass of the following compound (B-2) was used instead of the compound (A).

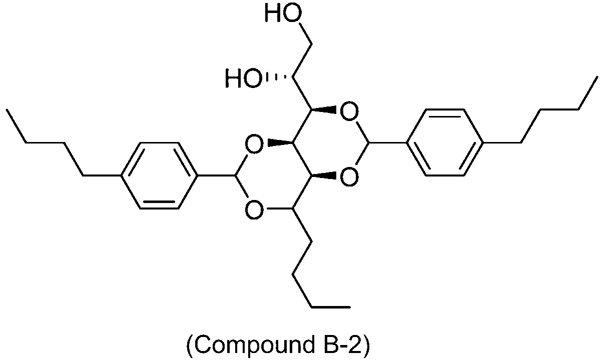

### (Comparative Example B-2-2)

A formed article was produced in the same manner as in Example 6, except that 0.8 parts by mass of the above compound (B-2) was used instead of the compound (A).

### <Evaluation of formed article>

### [Measurement of yellowing index (YI)]

The yellowing index (YI) of the obtained formed article was measured by using a haze meter (NIPPON DENSHOKU INDUSTRIES Co., Ltd., COH7700). The result was shown in the following Table. A smaller yellowing index (YI) means superior optical properties. It was found from the Table that the formed article of each Example had a YI value of 3 or less and therefore yellowing was suppressed.

**[Table 2]**

| | Compound | Amount added (parts by mass) | Yellowing index (YI) |
|---|---|---|---|
| Example 6 | Compound (A) | 0.5 | 2.71 |
| Example 7 | Compound (A) | 0.8 | 2.96 |
| Example 8 | Compound (B) | 0.5 | 2.94 |
| Example 9 | Compound (B) | 0.8 | 2.92 |
| Example 10 | Compound (C) | 0.5 | 2.61 |
| Example 11 | Compound (D) | 0.5 | 2.52 |
| Example 12 | Compound (E) | 0.5 | 2.96 |
| Comparative Example A-2-1 | Compound (A-2) | 0.5 | 3.23 |
| Comparative Example A-2-2 | Compound (A-2) | 0.8 | 4.30 |

### [Measurement of haze value]

The haze value of the obtained formed article was measured by using a haze meter (NIPPON DENSHOKU INDUSTRIES Co., Ltd., COH7700). The result was shown in the following Table. A smaller haze value (%) means superior optical properties. It was found from the Table that the formed article of each Example had a haze value of 11% or less and therefore the formed article was excellent in the transparency.

**[Table 3]**

| | Compound | Amount added (parts by mass) | Haze value (%) |
|---|---|---|---|
| Example 6 | Compound (A) | 0.5 | 8.18 |
| Example 7 | Compound (A) | 0.8 | 5.39 |
| Example 8 | Compound (B) | 0.5 | 10.7 |
| Example 9 | Compound (B) | 0.8 | 5.98 |
| Example 12 | Compound (E) | 0.5 | 8.89 |
| Comparative Example 3 | - | 0 | 58.84 |
| Comparative Example 4 | Compound (X) | 0.2 | 12.21 |
| Comparative Example 5 | Compound (Y) | 0.2 | 39.31 |
| Comparative Example A-2-1 | Compound (A-2) | 0.5 | 27.40 |
| Comparative Example A-2-2 | Compound (A-2) | 0.8 | 19.88 |

### [Measurement of fish eye]

The number of fish eyes in the obtained formed article was measured in accordance with the following method. The result was shown in the following Table. It was found that defects in appearance did not occur in the formed article of each Example. Defects in appearance did not occur even when the amount of the compound added was increased from 0.5 parts by mass to 0.8 parts by mass.

### Measurement method for fish eye

(1) In a test piece of the formed article (diameter: 30.0 mm), a cloudy part having a maximum length of 50 µm or more resulting from an undispersed nucleating agent was determined to be a fish eye, which was subjected to measurement (KEYENCE CORPORATION, digital microscope VHX-7100, observed at a magnification of 50x).
(2) In the case of a small number of fish eyes, the number of fish eyes on one test piece was counted. In the case of a large number of fish eyes, the number of fish eyes per an observation visual range was counted and then the number of fish eyes was multiplied by the value of [Area of test piece/Area of observation visual range].
(3) The process of (2) was performed using three test pieces produced and the average value of the number of fish eyes per one test piece was determined.
(4) With the number of fish eyes per one test piece of 10 or less, it was evaluated that defects in appearance did not occur in the formed article and the compound (I) was homogeneously dissolved and/or dispersed in the polyolefin resin.

**[Table 4]**

| | Compound | Amount added (parts by mass) | Fish eyes (number) |
|---|---|---|---|
| Example 6 | Compound (A) | 0.5 | 7 |
| Example 7 | Compound (A) | 0.8 | 9 |
| Example 8 | Compound (B) | 0.5 | 6 |
| Example 9 | Compound (B) | 0.8 | 10 |
| Comparative Example 4 | Compound (X) | 0.2 | 24 |
| Comparative Example 5 | Compound (Y) | 0.2 | 15 |
| Comparative Example A-1-1 | Compound (A-1) | 0.5 | 371 |
| Comparative Example A-1-2 | Compound (A-1) | 0.8 | 143 |
| Comparative Example A-2-1 | Compound (A-2) | 0.5 | 171 |
| Comparative Example A-2-2 | Compound (A-2) | 0.8 | 334 |
| Comparative Example B-1-1 | Compound (B-1) | 0.5 | 182 |
| Comparative Example B-1-2 | Compound (B-1) | 0.8 | 697 |
| Comparative Example B-2-1 | Compound (B-2) | 0.5 | 155 |
| Comparative Example B-2-2 | Compound (B-2) | 0.8 | 334 |

The entire contents of the description in Japanese Patent Application No. 2023-023452, filed on February 17, 2023, are herein incorporated by reference.

All of the patent literatures, the patent applications, and the technical standards described herein are herein incorporated by reference to the same extent as in a case where it is described specifically and individually that each of the literatures, patent applications, and technical standards is incorporated by reference.

## Claims

1. A compound represented by the following formula (I): 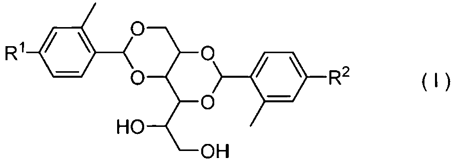 wherein R¹ and R² each independently represent an alkyl group having 2 to 5 carbon atoms.

2. The compound according to claim 1, wherein R¹ and R² each independently represent ethyl, n-propyl, i-propyl, n-butyl, or i-butyl.

3. A composition comprising the compound according to claim 1 or 2.

4. The composition according to claim 3, wherein the composition is used as a nucleating agent or a clarifying agent.

5. A formed article obtained by mixing the compound according to claim 1 or 2 or the composition according to claim 3, and a polyolefin resin.

6. A method for producing a transparent formed article, comprising mixing the compound according to claim 1 or 2 or the composition according to claim 3, and a polyolefin resin.

7. A method for producing a compound represented by the following formula (I), comprising reacting a benzaldehyde (α) having an alkyl group having 2 to 5 carbon atoms located at a para position and a methyl group located at an ortho position with a polyol in the presence of an acid catalyst: 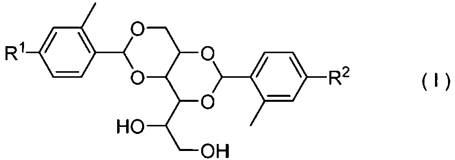 wherein R¹ and R² each independently represent an alkyl group having 2 to 5 carbon atoms.

8. The production method according to claim 7, further comprising reacting a benzaldehyde (β) having an alkyl group having 2 to 5 carbon atoms located at an ortho position and a methyl group located at a para position,
wherein a content of the benzaldehyde (β) based on a total amount of the benzaldehyde (α) and the benzaldehyde (β) is 50 mass% or less.
